# EUROPEAN PATENT APPLICATION

(11) **EP 4 728 864 A1**
(43) Date of publication of application: **22.04.2026**
(21) Application number: 24823531.9
(22) Date of filing: 28.03.2024
(51) Int. Cl.: A01N 1/02, C12N 5/0775, C12N 5/0735, C12N 5/074

(54) **COMPOSITION FOR INCREASING STABILITY AFTER THAWING OF FROZEN STEM CELL FORMULATIONS**

(30) Priority: 15.06.2023 KR 20230076841
(71) Applicant: Ra, Jeong Chan, Osong-eup, Heungdeoek-gu Cheongju-si, Chungcheongbuk-do 28166 (KR)
(72) Inventor: KIM, Eun-Young, Seoul 08529 (KR); RA, Jeong Chan, Cheongju-si Chungcheongbuk-do 28166 (KR)
(74) Representative: Mewburn Ellis LLP
(86) International application number: PCT/KR2024/003953
(87) International publication number: WO 2024/258010

(57) **Abstract**

The present invention relates to a composition (frozen formulation) used for cryopreservation of stem cells, and a cryopreservation method of stem cells using the composition. According to the cryopreservation solution of stem cells and the cryopreservation method using same of the present invention, the stem cells can be preserved for a long period of time, exhibit a high survival rate after being thawed, and can be directly applied to a living body without additional treatment after thawing.

## Description

### [Technical Field]

The present invention relates to a composition for use in cryopreservation of stem cells (cryopreservation formulation) and a method of cryopreserving stem cells using the composition.

### [Background Art]

The problem or limitation of currently-used stem cell refrigerated formulations is that the shelf life thereof is short, within 3 to 10 days, which causes distribution problems. To overcome this, there is an urgent need for development of a formulation that may be stored for a long time (specifically, for at least a year or even several years) without safety issues by extending the stability period of pure and highly effective stem cell therapies, and that may be administered to humans directly after thawing without further treatment when the patient needs the same while maintaining the stability and efficacy of the product. However, a stem cell frozen formulation must satisfy safety, stability, and efficacy requirements.

Although various cryopreservation compositions for stem cell storage have been developed, only one frozen injectable formulation has been commercialized for direct administration to humans, namely 'Prochymal', a stem cell therapy developed by Osiris for the treatment of graft-versus-host disease (GVHD). However, 10% DMSO is included in the excipient composition of 'Prochymal'.

Efforts have been made to prepare a composition for cell cryopreservation using recombinant human serum albumin, etc. (Korean Patent No. 10-1407355), and there have been attempts to use human serum instead of fetal bovine serum (Transplantation. 2000 Dec 27; 70(12):1780-7.). However, problems have arisen in that the original characteristics of stem cells are altered, for example by reduced or abnormally enhanced proliferation or differentiation potential of stem cells.

Currently, the most widely used cell cryoprotectant is DMSO (dimethyl sulfoxide). DMSO is essential for freezing cells, but concentrations exceeding 5% are potentially harmful to tissues. In this regard, 'Prochymal' may also be considered to have safety issues. Hence, it is necessary to reduce the DMSO content, and a stem cell frozen formulation in which stability and efficacy are maintained even with reduced DMSO content needs to be developed.

Accordingly, the present inventors have made great efforts to develop Cryopreserved Stem Cell Formulationin which the stability and efficacy of stem cells are maintained even at low DMSO concentrations, and ascertained that, when glucose is added to a cryopreservation composition for stem cell therapy (cryopreservation formulation), a cryopreservation formulation with reduced DMSO content may be prepared that maintains efficacy, ensures safety for human use, and exhibits high product stability, thus culminating in the present invention.

### [Disclosure]

An object of the present invention is to provide a composition for use in cryopreservation of stem cells having high stem cell storage stability and efficacy even at low DMSO concentration.

Another object of the present invention is to provide a method of cryopreserving stem cells using the cryopreservation formulation described above.

In order to accomplish the above objects, the present invention provides a composition for use in cryopreservation of stem cells containing glucose, DMSO, and human serum as active ingredients.

The present invention also provides a method of cryopreserving stem cells, including storing and freezing stem cells in the cryopreservation formulation described above.

### [Brief description of Drawings]

FIG. 1 shows results of microscopic examination of the viability of stem cells in the Ad-MSC fresh group and Ad-MSC frozen formulation group by staining with Trypan blue;
FIG. 2 shows FACS results of the surface antigens of stem cells in the Ad-MSC fresh group;
FIG. 3 shows FACS results of the surface antigens of stem cells in the Ad-MSC frozen formulation group;
FIG. 4 shows results of Oil-Red O staining after inducing differentiation of stem cells in the Ad-MSC fresh group and Ad-MSC frozen formulation group into adipocytes;
FIG. 5 shows results of Alizarin Red S staining after inducing differentiation of stem cells in the Ad-MSC fresh group and Ad-MSC frozen formulation group into osteocytes;
FIG. 6 shows results of inducing chondrogenic differentiation of stem cells in the Ad-MSC fresh group and Ad-MSC frozen formulation group;
FIG. 7 shows results confirming the attachment ability of stem cells in the Ad-MSC fresh group and Ad-MSC frozen formulation group after 8 hours of adherent culture; and
FIG. 8 shows results of the sterility test (direct method) of the Ad-MSC fresh group (A) and the Ad-MSC frozen formulation group (B).

### [Mode for Invention]

Unless otherwise defined, all technical and scientific terms used herein have the same meanings as typically understood by those skilled in the art to which the present invention belongs. In general, the nomenclature used herein is well known in the art and is typical.

DMSO (dimethyl sulfoxide), currently the most widely used cell cryoprotectant, is essential for freezing cells, but if the concentration exceeds 5%, DMSO may be harmful to tissues, so there is a need to reduce content thereof. The material that the present inventors found to make this possible is glucose. The idea of using glucose was derived from hibernating animals, in which glucose serves as a crucial factor in surviving freezing conditions. Glucose travels through the blood vessels to major organs and muscles and enters the cells. This series of mechanisms prevents cellular freezing and enables the body to resume normal function after thawing, without cellular damage, as before hibernation. This means that glucose is synthesized and used as a cryoprotectant. Furthermore, glucose is a safe material for the human body.

In the present invention, a cryopreservation composition for stem cell therapy (cryopreservation formulation) was developed that includes glucose with reduced DMSO content, thereby maintaining efficacy and also ensuring safety for human use and enhancing product stability. Of particular importance is not only maintaining freeze stability, but also ensuring stability and viability after thawing. The present inventors focused on this aspect in developing the formulation.

As used herein, the term "cryopreservation" means maintaining cells or tissues stably for a long period of time through freezing. Cells generally mutate at a rate of about one in ten thousand in culture, and if cell passages continue over a long period of time, the cell population changes into a different cell population from the original cell population, and in severe cases, a specific function of the cells may be lost due to passage culture. Also, infection with mycoplasma, etc. may occur during subculture. Due to these problems, cells or tissues are frozen to preserve the intrinsic characteristics thereof before they are lost, so that they may be taken out and used when needed, or tissues are cryopreserved. In particular, in order to use stem cells as a therapy, healthy stem cells must be available immediately when needed, so a method of effectively cryopreserving stem cells is particularly necessary.

Accordingly, an aspect of the present invention relates to a composition for use in cryopreservation of stem cells (cryopreservation formulation), containing glucose, DMSO, and human serum as active ingredients.

In the cryopreservation formulation of the present invention, the glucose may be contained at a concentration of 1 to 4% (v/v), preferably at a concentration of 1.5 to 3%, more preferably at a concentration of 1.5 to 2.5%.

In the cryopreservation formulation of the present invention, the DMSO may be contained at a concentration of 2 to 5% (v/v), preferably at a concentration of 3 to 4%.

In the cryopreservation formulation of the present invention, the human serum may be contained at a concentration of 91 to 97% (v/v).

A stem cell therapy frozen formulation according to the present invention may be administered not only by local injection (intra-articular, intramuscular, subcutaneous injection) but also intravenously.

Another aspect of the present invention relates to a method of cryopreserving stem cells, including storing and freezing stem cells in the cryopreservation composition described above.

In the present invention, the stem cells may be mesenchymal stem cells, embryonic stem cells, or induced pluripotent stem cells, but are not limited thereto.

The cryopreservation period of stem cells using the cryopreservation composition of the present invention may be used without limitation, but is preferably 1 day to 20 years, more preferably 3 days to 10 years, even more preferably 6 months to 3 years, but is not limited thereto.

In the present invention, it was confirmed that the cryopreservation formulation containing 2% glucose exhibited the viability of stem cells after thawing similar to or higher than that of the control group even when the concentration of DMSO, which is harmful to cells, was reduced.

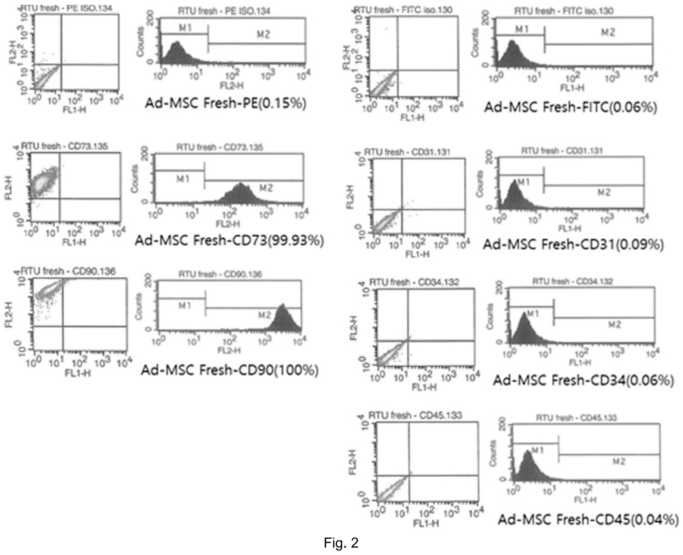

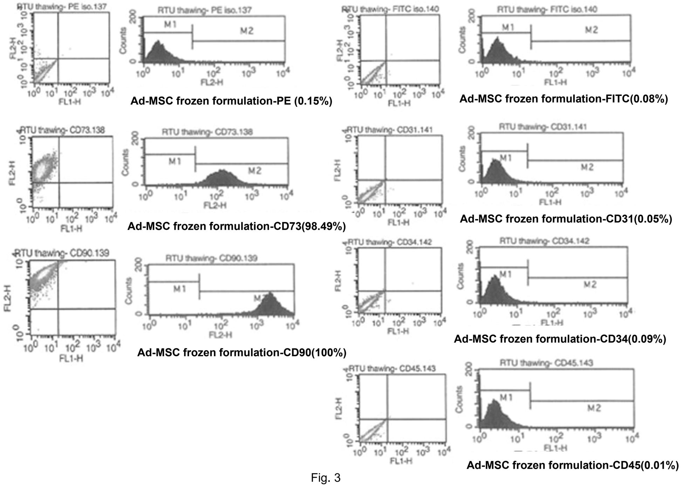

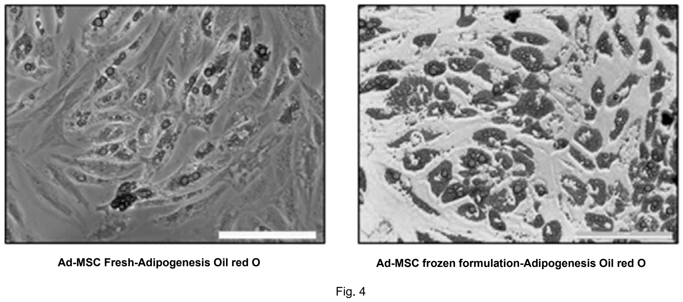

Also, in an embodiment of the present invention, it was confirmed that adipose-derived mesenchymal stem cells (Ad-MSCs) thawed after storage using the cryopreservation formulation containing glucose with reduced DMSO content (95% human serum + 2% glucose + 3% DMSO) exhibited high viability, expressed stem cell surface antigens (FIGs. 2 and 3), and maintained differentiation potential into adipocytes, osteocytes, and chondrocytes (FIGs. 4, 5, and 6).

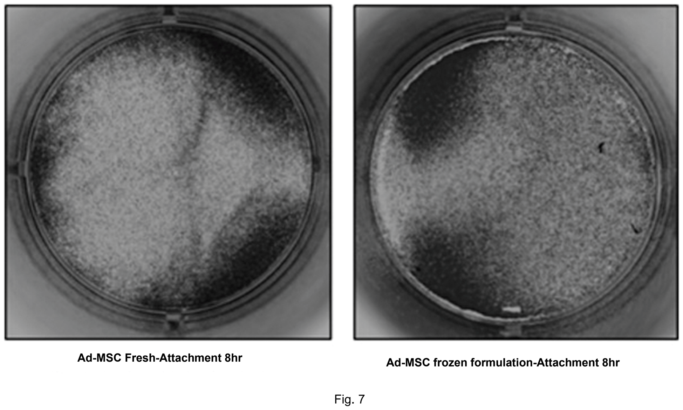

In another embodiment of the present invention, it was confirmed that the attachment ability of adipose-derived mesenchymal stem cells (Ad-MSCs) thawed after storage using the cryopreservation formulation of the present invention (95% human serum + 2% glucose + 3% DMSO) was well maintained (FIG. 7).

In still another embodiment of the present invention, it was confirmed that adipose-derived mesenchymal stem cells (Ad-MSC) thawed after storage using the cryopreservation formulation of the present invention (95% human serum + 2% glucose + 3% DMSO) maintained a sterile state, were mycoplasma negative, and had endotoxin content lower than the allowable level for injectable therapies (Example 5).

### [Examples]

A better understanding of the present invention may be obtained through the following examples. These examples are merely set forth to illustrate the present invention and are not to be construed as limiting the scope of the present invention, as will be apparent to those skilled in the art.

### Example 1: Preparation of glucose-containing cryopreservation solution and validation of efficacy/stability

After culturing stem cells and harvesting the cells, 1x10⁷ cells were aliquoted, suspended in 1 ml of solution for each group, and then placed in cryovials and frozen. The composition of the cryopreservation solution by group is as follows.

**[Table 1]**

| Group of Experiment 1 | |
|---|---|
| control | DMSO 5%+HS 95% |
| G1 | GLU 5% + HS 95% |
| G2 | DMSO 1% + HS 95% + GLU 4% |
| G3 | DMSO 2% + HS 95% + GLU 3% |
| G4 | DMSO 2.5% + HS 95% +GLU 2.5% |
| G5 | DMSO 3% + HS 95% + GLU 2% |
| G6 | DMSO 4% + HS 95% + GLU 1% |

| | |
|---|---|
| [Terminology: GLU: glucose, DMSO: dimethyl sulfoxide, HS: human serum] | |

The purpose of developing the cryopreservation solution of the present invention is to maintain the viability by minimizing the DMSO content as much as possible, and 5% DMSO, a concentration known to be safe for the body, was used as a control.

**[Table 2]**

| Group of Experiment 2 | |
|---|---|
| control | DMSO 5%+HS 95% |
| G1 | GLU 2% + DMSO 4% + HS 94% |
| G2 | GLU 2% + DMSO 3% + HS 95% |
| G3 | GLU 2% + DMSO 2.5% + HS 95.5% |
| G4 | GLU 2% + DMSO 2% + HS 96% |

In the experiment, the glucose (GLU) concentration was fixed at 2%, while the DMSO concentration was varied. After freezing, the samples were stored in a deep freezer for 3 to 10 days and then thawed, and the viability was measured over time to determine the optimal doses of DMSO and glucose. (After thawing, the viability was determined over time by storing the samples in a refrigerated state)

The results are shown below.

**[Table 3]**

| Results of Experiment 1 (0-6 hours after thawing, viability before freezing: 89%) | | | | | | | | |
|---|---|---|---|---|---|---|---|---|
| Time after group thawing | | Viability(%) | | | | | | |
| | | 0hr | 1hr | 2hr | 3hr | 4hr | 5hr | 6hr |
| cont | DMSO 5%+HS 95% | 87 | 82.2 | 88.1 | 76 | 75.7 | 72.7 | 79.3 |
| G1 | GLU 5%+HS 95% | 57.6 | 19.6 | 19 | 21.3 | 18.8 | 26.5 | 15.9 |
| G2 | DMSO 1%+4%GLU+HS | 76.9 | 52.4 | 48.4 | 56 | 52.8 | 55.5 | 45.8 |
| G3 | DMSO 2%+ 3%GLU+HS | 90.6 | 89 | 83 | 81.5 | 84.1 | 80.2 | 79.9 |
| G4 | DMSO 2.5%+2.5%GLU+HS | 91.7 | 92.9 | 85.4 | 88.3 | 88.6 | 91.2 | 89.5 |
| G5 | DMSO 3%+ 2%GLU+HS | 93.8 | 93.3 | 90.1 | 90.6 | 92.5 | 89.4 | 81.9 |
| G6 | DMSO 4%+1%GLU+HS | 87.5 | 90.9 | 84.2 | 90.1 | 91.2 | 92.2 | 89.4 |

**[Table 4]**

| Results of Experiment 1 (7-120 hours after thawing, viability before freezing: 89%) | | | | | | | | |
|---|---|---|---|---|---|---|---|---|
| Time after group thawing | | Viability(%) | | | | | | |
| | | 7hr | 8hr | 24hr | 33hr | 48hr | 57hr | 120hr |
| cont | DMSO 5%+HS 95% | 81.2 | 82.4 | 80.3 | 79.5 | 78.1 | 68.2 | 69.9 |
| G1 | GLU 5%+HS 95% | 19.7 | 20 | 19.3 | 16.8 | 16.3 | 14.6 | 10.8 |
| G2 | DMSO 1%+4%GLU+HS | 47.8 | 46 | 79.1 | 43.5 | 43.9 | 30.5 | 31.7 |
| G3 | DMSO 2%+ 3%GLU+HS | 81.5 | 81 | 84.3 | 77.4 | 75.7 | 58.9 | 42 |
| G4 | DMSO 2.5%+2.5%GLU+HS | 87.3 | 84.3 | 92.2 | 79.1 | 84.1 | 68.2 | 62.1 |
| G5 | DMSO 3%+ 2%GLU+HS | **92.1** | **91.1** | **89.3** | **90.4** | **81.5** | **80** | **68.5** |
| G6 | DMSO 4%+1%GLU+HS | 92.8 | 88.8 | 86.7 | 90.3 | 78.8 | 78.8 | 75.3 |

**[Table 5]**

| Results of Experiment 2 (0-6 hours after thawing, viability before freezing: 92.9%) | | | | | | | | |
|---|---|---|---|---|---|---|---|---|
| Time after group thawing | | Viability(%) | | | | | | |
| | | 0hr | 1hr | 2hr | 3hr | 4hr | 5hr | 6hr |
| cont | DMSO 5%+HS | 95.9 | 94.1 | 88.7 | 94.7 | 90.2 | 89.7 | 89.2 |
| G1 | GLU 2%+DMSO 4%+HS | 92.7 | 90.6 | 93.5 | 86.4 | 87.6 | 87.1 | 87 |
| G2 | GLU 2%+DMSO 3%+HS | 94.8 | 90.4 | 91.9 | 90.3 | 93 | 88.6 | 83.8 |
| G3 | GLU 2%+DMSO 2.5%+HS | 93.6 | 83.7 | 85.6 | 85.6 | 87.1 | 88.7 | 85.1 |
| G4 | GLU 2%+DMSO 2%+HS | 90.5 | 82.5 | 81.5 | 80.4 | 78.3 | 73.6 | 74.6 |

**[Table 6]**

| Results of Experiment 2 (7-48 hours after thawing, viability before freezing: 92.9%) | | | | | | |
|---|---|---|---|---|---|---|
| | Time after group thawing | Viability(%) | | | | |
| | | 7hr | 8hr | 24hr | 32hr | 48hr |
| cont | DMSO 5%+HS | 86.3 | 78.4 | 89.3 | 83.9 | 80.4 |
| G1 | GLU 2%+DMSO 4%+HS | 87.8 | 83.3 | 84.6 | 87.1 | 85 |
| G2 | GLU 2%+DMSO 3%+HS | **89.1** | **91.4** | **85.8** | **90.3** | **83.2** |
| G3 | GLU 2%+DMSO 2.5%+HS | 83.4 | 78.2 | 71.1 | 79.2 | 66.7 |
| G4 | GLU 2%+DMSO 2%+HS | 70.2 | 65.6 | 70.5 | 58.2 | 50.9 |

For the cryopreservation formulation containing 2% glucose, it was confirmed that the viability of stem cells after thawing was similar to or higher than that of the control group even when the DMSO concentration was reduced.

### Example 2: Characterization of stem cells after cryopreservation in frozen formulation

After culturing and harvesting adipose-derived stem cells (Ad-MSCs), the cells were suspended in the cryopreservation formulation (95% human serum + 2% glucose + 3% DMSO) according to the present invention, placed in a cryogenic container, frozen in a deep freezer, and after 9 days, thawed in a 37°C water bath, and then tested for stem cell viability, stem cell characteristics, efficacy, activity, sterility (direct method), mycoplasma detection (RT-PCR), and endotoxin levels. Through the above experiments, the cytotoxicity, stem cell characteristics, efficacy, activity, and safety of the Ad-MSC frozen formulation were determined, confirming that stem cells frozen using the cryopreservation formulation of the present invention retained safety and efficacy, allowing direct application as a cell therapy after thawing without further treatment.

**[Table 7]**

| Experimental groups and conditions | |
|---|---|
| Group | Conditions |
| Ad-MSC fresh | Ad-MSCs were cultured, harvested, and used |
| Ad-MSC frozen formulation | Ad-MSCs were cultured and harvested, suspended in a cryopreservation formulation (95% human serum + 2% glucose + 3% DMSO), placed in a cryogenic container, and frozen in a deep freezer for a certain period. After 9 days, the cells were thawed and used |

### 2-1: Determination of stem cell viability

The viability of the Ad-MS fresh group and Ad-MSC frozen formulation group was assessed using Trypan blue.

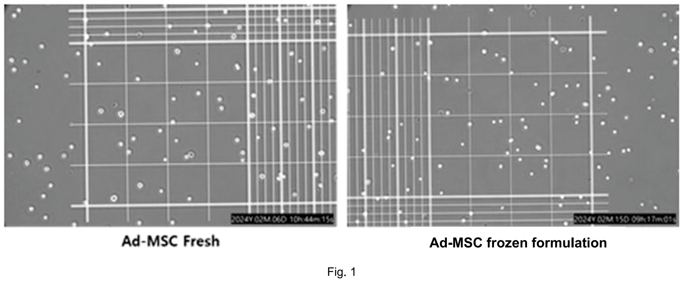

Therefore, as shown in FIG. 1 and Table 8, both groups exhibited viability of 90% or more. In conclusion, it was confirmed that the frozen formulation exhibited no cytotoxicity after thawing.

**[Table 8]**

| Viability of Ad-MSC fresh & Ad-MSC frozen formulation | | |
|---|---|---|
| Viability (%) | | |
| No. | Ad-MSC fresh | Ad-MSC frozen formulation |
| 1 | 95.6 | 89.7 |
| 2 | 94.0 | 91.9 |
| 3 | 96.0 | 91.4 |
| 4 | 96.1 | 93.6 |
| 5 | 94.6 | 91.3 |
| 6 | 92.3 | 89.6 |
| SD | 1.5 | 1.5 |
| Average (%) | 94.7 | 91.2 |

### 2-3: Identification of Ad-MSC surface antigens

After reacting the Ad-MS fresh and Ad-MSC frozen formulation with stem cell surface antigen CD markers, surface antigens were identified using FACS.

Therefore, as shown in FIGs. 2 and 3 and Table 9, the characteristics of surface antigens of adipose-derived mesenchymal stem cells (positive markers: CD73, CD90/negative markers: CD31, CD34, CD45) were observed in both groups.

**[Table 9]**

| FACS results of Ad-MSC fresh & Ad-MSC frozen formulation | | |
|---|---|---|
| CD marker | Ad-MSC fresh (%) | Ad-MSC frozen formulation (%) |
| Human CD73 | 99.93 | 98.49 |
| Human CD90 | 100 | 100 |
| Human CD31 | 0.09 | 0.05 |
| Human CD34 | 0.06 | 0.09 |
| Human CD45 | 0.04 | 0.01 |

| | | |
|---|---|---|
| *positive % | | |

### 2-3: Evaluation of differentiation potential of Ad-MSCs (adipogenesis, osteogenesis, and chondrogenic differentiation)

To assess the differentiation potential of Ad-MSCs into adipocytes, stem cells in the Ad-MSC fresh group and Ad-MSC frozen formulation group were seeded in 12-well culture dishes, and adipogenesis was induced using the StemPro^{™} Adipogenesis Differentiation Kit (Gibco, A1007001), followed by Oil Red-O staining to assess adipogenesis.

Therefore, as shown in FIG. 4, it was confirmed that adipogenesis was induced in both groups.

To assess the differentiation potential of Ad-MSCs into osteocytes, stem cells in the Ad-MSC fresh group and Ad-MSC frozen formulation group were seeded in 12-well culture dishes, and osteogenesis was induced using the StemPro^{™} Osteogenesis Differentiation Kit (Gibco, A1007201), followed by Alizarin Red staining to assess osteogenesis.

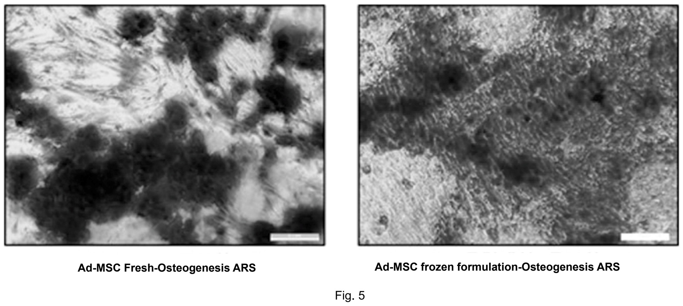

Therefore, as shown in FIG. 5, it was confirmed that osteogenesis was induced in both groups.

To assess the differentiation potential of Ad-MSCs into chondrocytes, stem cells in the Ad-MSC fresh group and Ad-MSC frozen formulation group were seeded in Falcon tubes, and TGF-β3 (Lonza, PT-4124) was added to Human Mesenchymal Stem Cell (hMSC) Chondrogenic Differentiation Medium BulletKit^{™} (Lonza, PT-3003) to induce chondrogenic differentiation.

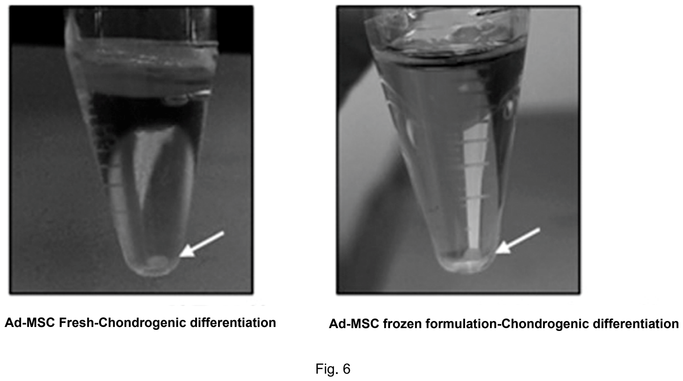

Therefore, as shown in FIG. 6, it was confirmed that chondrogenic differentiation was successfully induced in a spherical form in both groups.

### Example 3: Evaluation of therapeutic efficacy of stem cells after cryopreservation in frozen formulation

To assess changes in the efficacy of mesenchymal stem cells after cryopreservation in the frozen formulation, whether the level of a cartilage regeneration factor was maintained was determined.

After inducing chondrogenic differentiation using chondrogenic induction medium, successful spherical chondrogenic differentiation was confirmed. The level of human TSP-2, a cartilage regeneration factor, was determined using Elisa assay (Human TSP-2 Elisa Kit (R&D Systems, DTSP20)).

Therefore, as shown in Table 10, the Ad-MSC frozen formulation group expressed TSP-2 in a similar level to that of the cartilage regeneration factor (TSP-2) expressed in the Ad-MSC fresh group, confirming that the efficacy of stem cells was stably maintained even during preservation in the Ad-MSC frozen formulation.

**[Table 10]**

| Human TSP-2 concentration (pg/ml) produced in Ad-MSC fresh group and Ad-MSC frozen formulation group | |
|---|---|
| Group | Human TSP-2 (pg/ml) |
| Ad-MSC Fresh | 4464 pg/ml |
| Ad-MSC frozen formulation | 5776 pg/ml |

### Example 4: Evaluation of attachment ability of stem cells after cryopreservation in frozen formulation

To assess the activity of the Ad-MSC frozen formulation, the thawed stem cells were attached and the attachment ability thereof after 8 hours was determined.

Stem cells in the Ad-MSC fresh group and Ad-MSC frozen formulation group were seeded in 12-well culture dishes, and after 8 hours, the cells were fixed with 4% paraformaldehyde, followed by crystal violet staining to determine the extent of attachment.

Therefore, as shown in FIG. 7, the cells in both groups were well attached, confirming that the activity of stem cells was maintained even after cryopreservation using the Ad-MSC frozen formulation.

### Example 5: Evaluation of stability of stem cells after cryopreservation in frozen formulation

The aim was to assess the safety of stem cells after cryopreservation in the Ad-MSC frozen formulation, intended for direct application *in vivo* as a cell therapy. To this end, tests to confirm sterility were performed using the direct method and mycoplasma detection assay, and the absence of toxicity for human administration was verified using endotoxin assay (colorimetric method).

### 5-1: Sterility test (direct method)

Sterility testing was performed by introducing the sample into sterile medium followed by culture in a BOD incubator, and sterility was confirmed when no microbial growth was visually observed in the medium on the final day (14^{th} day) of the culture period (negative).

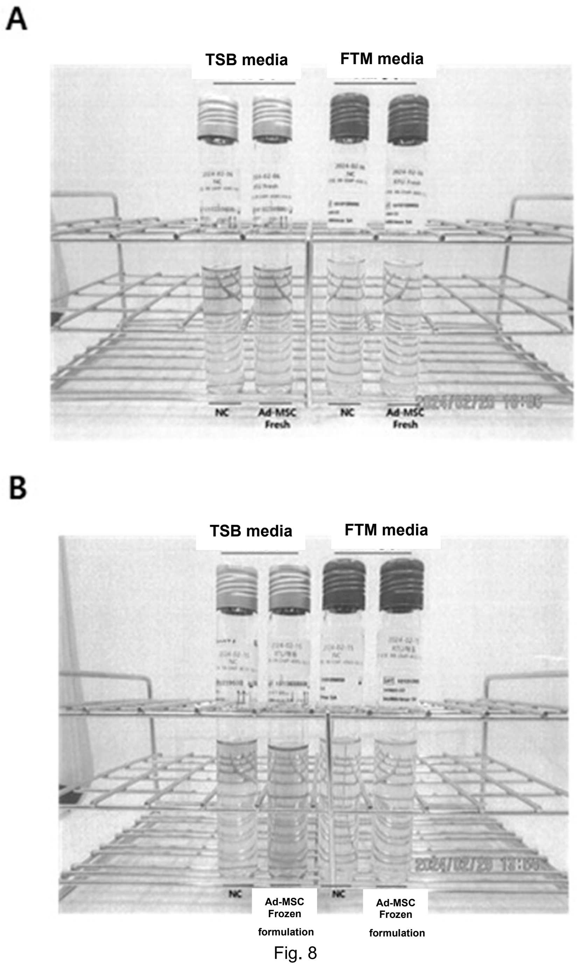

Based on results of the test using stem cells in the Ad-MSC fresh group and Ad-MSC frozen formulation group as samples, as shown in FIG. 8 and Table 11, both samples tested negative, confirming the safety of the samples.

**[Table 11]**

| Sterility test results of Ad-MSC fresh group and Ad-MSC frozen formulation group | |
|---|---|
| Sample | Results |
| Ad-MSC Fresh | Negative |
| Ad-MSC frozen formulation | Negative |

### 5-2: Mycoplasma detection assay (real-time PCR)

Stem cells in the Ad-MSC fresh group and Ad-MSC frozen formulation group were used as samples, and DNA of the samples was prepared and tested for mycoplasma detection assay using the MycoQSearch Mycoplasma qPCR Detection Kit (CellSafe, QDEP-100).

The criteria for the result judgment are that the Ct value of the HEX Channel, which is the PCR result of the internal DNA added to confirm that the test was performed properly, must be positive (Ct value < 40), and the Ct value of the FAM Channel, which is the PCR result of mycoplasma, must be negative (Ct value = undetermined).

Based on results of the mycoplasma detection assay (real-time PCR) of the Ad-MSC fresh group and Ad-MSC frozen formulation group, as shown in Tables 12 and 13, the PC (positive control) was positive, the NC (negative control) was negative, and the samples were negative, confirming the safety of the samples in both the Ad-MSC fresh group and the Ad-MSC frozen formulation group.

**[Table 12]**

| Mycoplasma detection assay results of Ad-MSC fresh group | | |
|---|---|---|
| Sample | Results | |
| | HEX Channel Ct value (Internal DNA) | FAM Channel Ct value (Mycoplasma PCR) |
| Positive control | 27.92 | 24.18 |
| Negative control | 29.35 | Undetermined |
| Ad-MSC fresh | 23.70 | Undetermined |

**[Table 13]**

| Mycoplasma detection assay results of Ad-MSC frozen formulation group | | |
|---|---|---|
| Sample | Results | |
| | HEX Channel Ct value (Internal DNA) | FAM Channel Ct value (Mycoplasma PCR) |
| Positive control | 28.76 | 23.98 |
| Negative control | 31.11 | Undetermined |
| Ad-MSC frozen formulation | 25.38 | Undetermined |

### 5-3: Endotoxin (colorimetric) assay

Endotoxin refers to lipopolysaccharide (LPS), which accounts for 70% of the cell wall components of Gram-negative bacteria, and is a pyrogenic substance that causes a fever reaction when infected with human blood. Therefore, all medicines administered to the human body must not exceed the endotoxin limit to be used as safe therapies.

The capacity of the Ad-MSC fresh group and Ad-MSC frozen formulation group is 1 ml, so when calculated using the endotoxin limit calculation method below, the endotoxin limit for intravenous and intramuscular injection is 349.6 EU/ml, and the endotoxin limit in intrathecal injection is 13.9 EU/ml (based on the United States Pharmacopeia (USP) in Table 14).

**[Table 14]**

| Endotoxin limit (EU/ml) according to the United States Pharmacopeia (USP) | | |
|---|---|---|
| Administratio n method | Calculation | Endotoxin limit (EU/ml) |
| Intramuscular or intravenous injection | Maximum dose per kg (standard adult body weight = 70 kg) = 1 ml/70kg = 0.0143 ml/kg | 349.6 EU/ml |
| | Endotoxin threshold = K/M = 5 | |
| | EU/Kg/0.0143 ml/kg = 349.6 EU/ml | |
| Intrathecal injection | Maximum dose per kg (standard adult body weight = 70 kg) = 1 ml/70kg = 0.0143 ml/kg | 13.9 EU/ml |
| | Endotoxin threshold = K/M = 0.2 | |
| | EU/Kg/0.0143 ml/kg = 13.9 EU/ml | |
| * Calculated based on the United States Pharmacopeia (USP), with the Korean standard applying a body weight of 60 kg | | |

Based on results of endotoxin assay in the Ad-MSC fresh group and Ad-MSC frozen formulation group, as shown in Table 15, the safety of the samples was proven as the endotoxin levels were determined to be much lower than the endotoxin limit.

**[Table 15]**

| Endotoxin assay results of Ad-MSC fresh group and Ad-MSC frozen formulation group | |
|---|---|
| Sample | Results |
| Ad-MSC Fresh | <1.00 EU/ml |
| Ad-MSC frozen formulation | <1.14 EU/ml |

### [Industrial Applicability]

According to the composition for cryopreservation of stem cells of the present invention and the cryopreservation method using the same, stem cells can be preserved for extended periods, exhibit high viability after thawing, and can be directly applied *in vivo* after thawing without further treatment, thereby achieving excellent effects.

Having described certain parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

## Claims

1. A composition for use in cryopreservation of stem cells containing glucose, DMSO, and human serum as active ingredients.

2. The composition according to claim 1, wherein the glucose is contained at a concentration of 1 to 4% (v/v).

3. The composition according to claim 1, wherein the DMSO is contained at a concentration of 2 to 5% (v/v).

4. The composition according to claim 1, wherein the human serum is contained at a concentration of 91 to 97% (v/v).

5. A method of cryopreserving stem cells, comprising storing and freezing stem cells in the composition according to any one of claims 1 to 4.

6. The method according to claim 5, wherein the stem cells are mesenchymal stem cells, embryonic stem cells, or induced pluripotent stem cells.
